# EUROPÄISCHE PATENTSCHRIFT

(11) **EP 1 337 236 B1**
(45) Veröffentlichungstag und Bekanntmachung des Hinweises auf die Patenterteilung: **04.08.2004**
(21) Anmeldenummer: 01994656.5
(22) Anmeldetag: 14.11.2001
(51) Int. Cl.: A61K 9/00, A61K 31/195, A61K 31/22, A61K 33/24, A61K 33/30

(54) **ENTERAL ZU VERABREICHENDES SUPPLEMENT ZUR PARENTERALEN ERNÄHRUNG ODER PARTIELLEN ENTERALEN/ORALEN ERNÄHRUNG BEI KRITISCH KRANKEN, CHRONISCH KRANKEN UND MANGELERNÄHRTEN**
SUPPLEMENT TO BE ENTERALLY ADMINISTERED FOR PARENTERAL NUTRITION OR PARTIAL ENTERAL/ORAL NUTRITION OF THE CRITICALLY ILL, THE CHRONICALLY ILL AND PEOPLE WITH MALNUTRITION
COMPLEMENT A ADMINISTRER PAR VOIE ENTERALE POUR NUTRITION PARENTERALE OU NUTRITION ENTERALE/ORALE PARTIELLE DANS LE CAS DE MALADIES CRITIQUES OU CHRONIQUES ET DE MALNUTRITION

(30) Priorität: 17.11.2000 DE 10057290
(43) Veröffentlichungstag der Anmeldung: 27.08.2003
(73) Patentinhaber: Fresenius Kabi Deutschland GmbH, 61352 Bad Homburg v.d.H. (DE)
(72) Erfinder: KESSLER, Barbara, 61476 Kronberg (DE); RIEDEL, Angelika, 61279 Grävenwiesbach (DE); SUCHNER, Ulrich, 85435 Erding (DE)
(74) Vertreter: Ackermann, Joachim, Dr.
(86) Internationale Anmeldenummer: PCT/EP2001/013163
(87) Internationale Veröffentlichungsnummer: WO 2002/039978

(56) Entgegenhaltungen:
- EP-A- 0 705 542
- EP-A- 0 756 827
- EP-A- 0 891 719
- WO-A-94/14458

## Beschreibung

Enteral zu verabreichendes Supplement zur parenteralen Ernährung oder partiellen enteralen/oralen Ernährung bei kritisch Kranken, chronisch Kranken und Mangelernährten

Die vorliegende Erfindung betrifft eine bei kritisch Kranken (Intensivpatienten oder operativen Patienten), chronisch Kranken oder Mangelernährten zusätzlich zu einer parenteralen oder unzureichenden oralen Ernährung, enteral zu verabreichende bzw. verabreichbare Ergänzungsnahrung (im Folgenden als Supplement bezeichnet), mit welcher schwere Störungen der Darmbarriere entweder im Sinne einer Protektion verhütet oder nach ihrem Eintreten therapiert werden sollen.

Die Darmbarriere umfasst im Sinne der Erfindung nicht nur die epithelialen Schichten der intestinalen Mukosa sondern auch die Mikroflora, die Peristaltik, die Mucusproduktion sowie die regionale Immunabwehr als Bestandteil des "gut associated lymphoid tissue" (GALT). Die Darmbarriere repräsentiert damit die erste Verteidigungslinie gegenüber einer drohenden Durchwanderung von pathogenen Keimen und Toxinen ― im Folgenden Translokation genannt. Die Bedeutung ihrer "Pflege" ist bereits im Zusammenhang mit der frühen enteralen Emährungstherapie des kritisch Kranken aufgezeigt worden.

Lebensbedrohliche Störungen anderer Organfunktionen, wie insbesondere von Herz, Lunge und Nieren, können heute durch Organersatz oder andere therapeutische Interventionen weitgehend kompensiert werden. Für den Darm ist dies nicht der Fall. Der "Darm" mit seinen lebenswichtigen Barrierefunktionen wurde folglich für die Prognose der oben benannten Patientengruppen vielfach zum kritischsten Organ.
Als kritisch Kranke oder Schwerstkranke im Sinne der Erfindung werden Patienten mit Krankheitsbildern angesehen, wie sie z. B. bei schweren Infektionen und Traumen, Polytraumen, akuten Strahlungsschäden, schweren Verbrennungen, aber auch bei großen Operationen auftreten, und die mit Störungen von Vitalfunktionen wie etwa der Atmung, der Kreislaufstabilität oder der Ausscheidungsfunktionen einhergehen. Als chronisch Kranke im Sinne der Erfindung werden Patienten mit benignen oder malignen chronisch konsumierenden Krankheitsbildern angesehen, die ohne Bedrohung der Vitalfunktionen verlaufen. Auch diese Patienten können häufig eine konventionelle enterale/orale Ernährung wegen mangelhafter Digestion und Assimilation nur unzureichend aufnehmen, wie z.B. bei chronisch entzündlichen Darmerkrankungen mit supprimiertem Immunsystem (Morbus Crohn oder Colitis ulcerosa) oder etwa Tumorpatienten. Als Mangelernährte im Sinne der Erfindung werden Patienten angesehen, die aus unterschiedlichsten Gründen nicht in ausreichendem Maße Nahrung zu sich nehmen können, wie zum Beispiel mangelemährte geriatrische Patienten.

In der initialen Versorgung schwer traumatisierter Patienten sind große Fortschritte erzielt worden. Eindrucksvoll wird dies mit einer Verminderung der Frühmortalität dieser Patienten dokumentiert. Die Spätmortalität schwer traumatisierter, kritisch kranker Patienten bleibt jedoch immer noch durch eine hohe Inzidenz von Sepsis und Multiorganversagen (MOV) belastet. Der Zusammenbruch der Darmbarriere des Gastrointestinaltraktes wurde als die wesentliche Eintrittstelle für Erreger und Toxine erkannt. Nach "Translokation" von lebenden Bakterien sowie von Toxinen durch die Darmwand in den Pfortaderkreislauf erfolgt konsekutiv deren Aufnahme auch in weitere Organe, wie die Leber und die Lunge. Insbesondere die Kupfferzellen des retikuloendothelialen Systems der Leber, sowie die Alveolarmakrophagen der Lunge, beteiligen sich an der Mediierung der sich dann entwickelnden Stressantwort. Sie sind als die Orte der Produktion entzündungsfördernder Mediatoren bekannt, wie etwa die Interleukine II-1, II-2, der Tumornekrosefaktor TNF-α und Prostaglandin E₂. Diese Mediatoren sind Ursache und Modulatoren einer klinisch imponierenden generalisierten Entzündungsreaktion, welche im Folgenden als "Systemic Inflammatory Response Syndrome" (SIRS) bezeichnet wird. Sehr vereinfacht wurden die Vorstellungen zur systemischen Inflammation viszeralen Ursprungs mit dem Leitsatz vom "Darm als dem Starter" und der "Leber als dem Motor" des Multiorganversagens zusammengefasst.

Neuen Erkenntnissen zufolge wirken sich emährungstherapeutische Faktoren, wie etwa Mangelemährungszustände oder eine fehlende enterale Ernährung, fördernd auf die Entwicklung bakterieller Translokationsphänomene aus. Dies gilt insbesondere für den kritisch Kranken, der durch rezidivierende intestinale Hypoperfusion und konsekutive Bildung von freien Radikalen während Reperfusion einem hohen Risiko einer Störung der Darmbarriere ausgesetzt ist. Es wird in zunehmendem Maße deutlich, daß enterale Ernährungsmaßnahmen einen wichtigen Beitrag zur Aufrechterhaltung von Struktur und Funktion der intestinalen Mukosa liefern können. Darüber hinaus gibt es eindeutige Hinweise dafür, daß sich durch eine enterale Substratzufuhr die Risiken einer bakteriellen Translokation sowie die sich aus ihr ergebenden systemischen Infektionen minimieren lassen. Die möglichen Vorteile der frühen enteralen Ernährung des kritisch Kranken lassen sich gegenwärtig in 4 Punkten zusammenfassen:
1. Die frühe enterale Emährung des kritisch Kranken trägt zur Protektion der intestinalen Barriere sowie zu deren Regeneration nach vorausge-gangenem mukosalem Trauma (bei z.B. viszeraler Hypoperfusion) und vermindert die Inzidenz von Infektionen sowie von Organdysfunktionen auf dem Boden eines reduzierten intestinalen Translokationsgeschehens.
2. Die posttraumatisch innerhalb von Stunden begonnene minimale enterale Ernährung führt zu einer verminderten Freisetzung von Stresshormonen und Mediatoren und damit zu einer verminderten Stressantwort, welche sich konsekutiv in einem geringeren Energieverbrauch und in einer verminderten Katabolie des Patienten auswirkt.
3. Die Wahl des physiologischeren enteralen Applikationsweges, der den viszeralen "first pass Effekt" mit einbezieht, hat eine Verbesserung der Substratverwertung und
4. eine Steigerung der Substratverträglichkeit zur Folge. Die Steigerung der Substratverwertung äußert sich neben einer günstigeren Substrat-Homöostase und einer Verbesserung der viszeralen Proteinsynthese letztlich in einer Optimierung des Emährungsstatus. Die Verbesserung der Substratverträglichkeit äußert sich klinisch in einer geringeren Rate an Nebenwirkungen. Dies betrifft insbesondere die viszeralen Organe wie Pankreas, Leber, Darm und macht sich in einem geringeren Anstieg der Integritätsparameter sowie in einer besser erhaltenen intestinalen Resorptionsleistung bemerkbar. Weiterhin wurde wiederholt eine Reduktion der Inzidenz gastrointestinaler Blutungen beobachtet.

Nach heutiger Ansicht darf daher die Ernährung kritisch Kranker nicht auf eine vollständige Umgehung des Intestinaltraktes abgestellt sein, so lange der Darm zumindest noch teilweise seine Funktionen übernehmen kann. Dies kann auch auf chronisch kranke Patienten - insbesondere solchen mit entzündlichen Darmerkrankungen - übertragen werden.

Kritisch Kranke ebenso wie chronisch kranke Patienten mit entzündlichen Darmerkrankungen weisen jedoch häufig eine nur unzureichende gastrointestinale Toleranz hoher Volumina konventioneller enteraler Nährlösungen auf. Dies gilt insbesondere für die bedarfsdeckenden Mengen an Kalorien- und Stickstoffträgern (Glucose, Fett, Aminosäuren). Die zur enteralen bedarfsdeckenden Verabreichung erforderlichen Volumina herkömmlicher Nährlösungen liegen typischerweise bei 1,5 - 2 Liter für eine Tagesdosis (Sondenernährung) und werden von diesen Patienten selbst dann kaum toleriert, wenn die Nährlösung über den jejunalen Applikationsweg verabreicht wird. Die gastrale Applikation ist häufig aufgrund von Magenentleerungsstörungen besonders problematisch.

Kritisch wie chronisch Kranke unterliegen zudem lange anhaltenden, schweren Belastungen des Stoffwechsels, dem Postaggressionssyndrom, welches bei erhöhtem Ruheenergiebedarf mit einer katabolen Stoffwechsellage verbunden ist. Dies führt zu einem erhöhten Bedarf an ausgewählten Makro- und Mikronährstoffen wie etwa Glutamin oder definierten Vitaminen und Spurenelementen. Ihr Mangel geht mit schweren Einschränkungen der Immun- und Organfunktionen einher und bedingt eine Erhöhung der Morbidität und Mortalität.

Die unzureichende Versorgung kritisch und chronisch Kranker mit definierten Nährstoffen zur Aufrechterhaltung oder Wiederherstellung der Darmbarriere basiert somit auf der unzureichend möglichen enteralen Zufuhr einerseits und dem erhöhten Bedarf dieser Substrate andererseits. Bei schwer mangeler-nährten Patienten kann es durch Substratdefizite und konsekutiver Störung der intestinalen Darmbarriere ebenfalls zur Translokation kommen, welche auch bei diesen Patienten zu einer systemischen Inflammation führen kann, die zwar graduell geringer ausgeprägt in Erscheinung tritt als bei kritisch und chronisch Kranken, die jedoch ebenso zu infektiösen Komplikationen führen kann.

Bis heute werden wichtige, die Darmbarriere schützende Substrate überwiegend auf parenteralem Wege zugeführt. Auf diesem Wege konnte jedoch nicht der erwünschte protektive und therapeutische Einfluß auf die Darmbarriere erzielt werden.

Enteral verabreichbare Substrate sind bereits beschrieben worden. So offenbart EP-A-875,155 ein peri-operatives Getränk, das neben Kohlehydraten Glutamin oder einen Glutamin-Vorläufer enthält. Aus WO-A-98/41,216 ist eine Zusammensetzung zur Prevention von hepatischer Steatosis bekannt, die neben einem Komplexbildner für Gallensäuren ein Immunonutrient enthalten kann.

Aus der WO-A-92/09277 ist eine oral oder parenteral verabreichbare Zusammensetzung bekannt, die neben freiem L-Glutamin wenigstens ein Derivat des L-Glutamins und gegebenenfalls wenigstens einen Vorläufer des L-Glutamins enthält.

Dieser Erfindung liegt die Aufgabe zugrunde, ein einem kritisch und chronisch Kranken oder einem Mangelernährten zusätzlich zu partieller oder vollständiger parenteraler Ernährung oder zusätzlich zu unzureichender enteraler/oraler Ernährung enteral zu verabreichendes oder verabreichbares Supplement zur Verfügung zu stellen, um die Darmbarrierefunktion aufrechtzuerhalten oder wiederherzustellen. Eine weitere Aufgabe besteht darin, ein der aktuellen Stresssituation angepaßtes Ernährungssystem aus parenteraler und enteraler Ernährung zur Verfügung zu stellen. Damit soll erreicht werden, dass bei kritisch wie chronisch Kranken oder mangeler-nährten Patienten dem drohenden "Darmversagen" entgegegewirkt, die Inzidenz von Infektionen und Organversagenszuständen verringert, die Mortalität reduziert und die Zeitdauer, in der eine parenterale Ernährung erforderlich ist, minimiert wird.

Generell werden diese Aufgaben dadurch gelöst, dass ein zusätzlich zu einer partiellen oder vollständigen parenteralen Ernährung oder zusätzlich zu einer unzureichenden enteralen/oralen Ernährung enteral zu verabreichendes Supplement zur Verfügung gestellt wird, das so konditioniert ist, dass a) die enteral verabreichten Substrate mit protektiv/therapeutischer Wirkung auf die Darmbarriere von der quantitativ zur Deckung des Energie und Eiweißbedarfs notwendigen Substratzufuhr losgelöst sind; b) das luminal" (enteral) erforderliche Angebot der protektiv/therapeutischen Substrate gesichert ist; c) die protektiv/therapeutischen Dosierungen sichergestellt sind; und d) die protektiv/therapeutischen Substrate im "Verbund", d.h. in einem definierten Mengenverhältnis gegeben werden, da ihre optimalen Wirkungen an die zeitgleiche adäquate Verfügbarkeit aller einzelnen Substrate geknüpft sind.

Im einzelnen werden diese Aufgaben dadurch gelöst, dass dem auf eine Tagesdosis bezogenen enteral zu verabreichenden Supplement in Kombination a) Glutamin und/oder Glutamin-Vorläufer in einer Menge im Bereich von 15 bis 70 g, b) mindestens zwei Vertreter aus der Gruppe der als Antioxidantien wirkenden Klasse und c) kurzkettige Fettsäuren und/oder Vorläufer kurzkettiger Fettsäuren, insbesondere Tributyrin, in einer Menge im Bereich von 0,5 bis 10 g zusammen mit ggf. weiteren, sich nach dem jeweiligen Krankheitsbild orientierenden Immuno/Pharmakonutrients zugesetzt werden.

Unter dem Begriff Glutamin-Vorläufer werden Verbindungen verstanden, die Glutamin enthalten, welches durch Stoffwechselaktivitäten freigesetzt wird. Beispiele für Glutamin-Vorläufer sind Derivate des Glutamins, wie Ester, Amide, N-alkyliertes Glutamin, Salze oder Keto-Vorläufer des Glutamins, wie α-Ketoglutarat, sowie kurzkettige glutaminhaltige Peptide, wie Di- bis Decapeptide, vorzugsweise Tripeptide, und ganz besonders bevorzugt Dipeptide. Beispiele für Tripeptide sind X-Gln-X', X-X'-Gln und X-Gln-Gln, wobei X und X' für natürlich vorkommende Aminosäuren stehen.

Bevorzugt sind die Tripeptide Ala-Gln-Ala, Ala-Ala-Gln, Ala-Gln-Gln, Gly-Gln-Gly, Gly-Gly-Gln, Gly-Gln-Gln, Ala-Gln-Gly, Gly-Gln-Ala, Ala-Gly-Gln und Gly-Ala-Gln.

Beispiele für Dipeptide sind X-Gln, wobei X für natürlich vorkommende Aminosäuren steht. Bevorzugt sind die Dipeptide Ala-Gln und Gly-Gln.

Mengenangaben beziehen sich bei Glutamin-Vorläufem auf den Glutamin-Anteil dieser Vorläufer.

Unter dem Begriff kurzkettige Fettsäuren sind Carbonsäuren mit zwei bis fünf, vorzugsweise zwei bis vier, Kohlenstoffatomen zu verstehen. Beispiele dafür sind Essigsäure, Propionsäure, α-Methylpropionsäure, Pentansäure (Valeriansäure) und insbesondere Buttersäure.

Unter dem Begriff Vorläufer kurzkettiger Fettsäuren werden Verbindungen verstanden, die kurzkettige Fettsäuren enthalten, welche durch Stoffwechselaktivitäten freigesetzt werden. Beispiele dafür sind Salze oder Ester kurzkettiger Fettsäuren. Die Ester können von ein- oder mehrwertigen Alkoholen abgeleitet sein. Beispiele für Ester sind Methyl- oder Ethylester, Phospholipide oder insbesondere Glycerinester. Ester mehrwertiger Alkohole können neben kurzkettigen auch mittel- oder langkettige Fettsäuren enthalten. Vorzugsweise leiten sich bei Estern mehrwertiger Alkohole mit unterschiedlichen Fettsäuren alle Säureteile von kurzkettigen Fettsäuren ab.

Bevorzugt werden Glycerinester kurzkettiger Fettsäuren, besonders bevorzugt wird Tributyrin (= Glycerintriester der Buttersäure). Mengenangaben beziehen sich bei Vorläufern kurzkettiger Fettsäuren auf den Anteil an kurzkettigen Fettsäuren dieser Vorläufer.

Unter dem Begriff Immuno-/Pharmakonutrients werden Nährstoff-komponenten verstanden, welche im täglichen Gesamtnährstoffangebot aus enteraler und ggf. parenteraler Verabreichung in höheren Konzentrationen enthalten sind, als dies der empfohlenen Tagesdosis gemäß RDA (Recommended Dietary Allowances) entspricht. Dieses Konzept einer zusätzlich zur parenteralen Ernährung oder zusätzlich zu einer partiellen oralen/enteralen Ernährung direkt auf die Darmmukosa und über die Pfortader auf die Leber einwirkenden Ernährung mit Immuno-/Pharmakonutrients dient dazu, den durch die Stresssituation lokal hervorgerufenen Konzentrationsabfall von Substraten und eine damit verbundene Einschränkung bzw. den Ausfall lebenswichtiger Stoffwechselfunktionen zu kompensieren, das Immunsystem und/oder gewisse biochemisch/physiologische Stoffwechselvorgänge zu unterstützen bzw. in vorteilhafter Weise zu regulieren und die Integrität der Darmbarriere zu sichern.

Unter dem Begriff Tagesdosis ist das für den jeweiligen Anwendungsfall zu verabreichende Volumen des erfindungsgemäßen Supplements zu verstehen. Typische Tagesdosen können bis zu 2000 ml betragen, liegen vorzugsweise im Bereich zwischen 200 bis 1000 ml, und ganz besonders bevorzugt bei 400 bis 500 ml.

Das erfindungsgemäße Supplement dient also für alle oben genannten Patientenkollektive, bei denen die Ernährung parenteral sicher gestellt werden muß oder nur teilweise enteral/oral durchgeführt werden kann, als eine enteral verabreichbare oder zu verabreichende Zusatznahrung. Mit dem erfindungsgemäßen Supplement wird jetzt eine entscheidende Verbesserung der Darmbarrierefunktionen und damit der Prognose der Patienten, soweit diese durch die Darmbarrierefunktionen bestimmt ist, ermöglicht. Die bisherige Praxis der frühen enteralen Ernährung bzw. enteralen Minimalernährung konnte diesen therapeutischen Beitrag nicht bringen, da sie im wesentlichen auf die quantitativ sichere Zufuhr von Energie und Eiweiß ausgerichtet war. Erst das erfindungsgemäße Supplement liefert in den notwendigen Konzentrationen und Mengenrelationen die Substrate, die zur Aufrechterhaltung von Struktur und Funktion schnell proliferierender Gewebe, wie der Mukosazellen oder der immunkompetenten Zellen, unverzichtbar sind. Es ist die hochdosierte enterale Zufuhr der nachstehend beschriebenen Substrate im erfindungsgemäßen Supplement, welche für die Aufrechterhaltung (Protektion) oder die Wiederherstellung (Therapie) der Darmbarriere die größte Bedeutung hat.

Erfindungsgemäß ist das enterale Supplement energiearm, während als Hauptquelle für die Zufuhr von Energie und Stickstoffträgern, Wasser und Elektrolyten, insbesondere am Beginn der künstlichen Ernährung eines kritisch Kranken, die parenterale Ernährung dient. Das enterale Supplement kann in einem geeigneten Lösungsmittel, vorzugsweise Wasser, gelöst entweder oral oder über eine im Gastrointestinalraum plazierte Sonde in einer Tagesdosis, die üblicherweise 1 Liter nicht überschreitet, verabreicht werden. Das erfindungsgemäße Supplement kann neben gelösten Bestandteilen auch dispergierte, suspendierte und/oder emulgierte Bestandteile enthalten.

Vorteilhafterweise wird am Beginn der Behandlung eines kritisch oder chronisch Kranken mit einem Supplementvolumen von 500 ml pro Tagesdosis begonnen. Mit schrittweiser Verbesserung des Allgemeinzustandes des Patienten können dann auch Energie- und Stickstoffträger zur Deckung des Kalorien- und Proteinbedarfs sowie Wasser und Elektrolyte zunehmend über den enteralen Applikationsweg zugeführt werden.

Die Substratzusammensetzung der Erfindung ist entsprechend der zugrunde gelegten Volumina unterschiedlich, wobei nachfolgend zwischen Supplement I (kleinere Volumina, wie 500 ml) und Supplement II (größere Volumina, wie 1000 ml) unterschieden wird.

### Supplement I:

Das enteral zu verabreichende erfindungsgemäße Supplement I weist neben Glutamin und/oder Glutamin-Vorläufer und kurzkettiger Fettsäure und/oder deren Vorläufer, wie Tributyrin, als essentielle Bestandteile noch mindestens zwei, vorzugsweise aber mehrere Antioxidantien bzw. antioxidativ wirksame Nährstoffe auf, welche z.B. Vitamin C, Vitamin E, S-Adenosylmethionin, Cystein, Cystin, Taurin, Glutathion, Selen, Zink, Polyphenole und Carotinoide, vorzugsweise β-Carotin, sein können, welche generell der Aufrechterhaltung der Darmbarriere dienen und welche insbesondere in synergistischer Weise zur Bekämpfung der während oxidativem Stress entstehenden freien Radikale herangezogen werden.

Vorteilhafterweise kann das erfindungsgemäße enterale Supplement noch Vorläufersubstanzen für die DNA- und RNA- Synthese aufweisen, vorzugsweise in Form von Nucleotiden. Darüber hinaus kann das erfindungsgemäße Supplement noch unter normalen Bedingungen aus Ballaststoffen von Mikroorganismen gebildete, den Metabolismus der Kolonozyten begünstigende Substrate, wie z.B. eine oder mehrere Substanzen mit calciumantagonistischer Wirkung, insbesondere Glycin aufweisen.

Dieses glutaminreiche Supplement mit den Antioxidantien und den kurzkettigen Fettsäuren und/oder deren Vorläufern, wie dem Tributyrin, dem bevorzugter-weise noch Nucleotide und/oder Glycin mit in den angegebenen Bereichsgrenzen liegenden Konzentrationen zugesetzt sind, ist dafür vorgesehen, Mangelernährten sowie kritisch oder chronischen Kranken am Behandlungsbeginn, zusätzlich zu einer vollständigen parenteralen Ernährung oder zusätzlich zu einer enteralen/oralen Ernährung, auf enteralem Wege verabreicht zu werden. Anstelle der genannten Substanzen können auch physiologisch gleich wirkende Verbindungen eingesetzt werden, z.B. α-Ketoglutarat für Glutamin.

Die Aufnahme des Supplements durch den Patienten kann entweder oral oder über eine Sonde erfolgen. Das vorzugsweise in Wasser gelöste Supplement ist vorzugsweise auf eine Tagesdosis portioniert und liegt in diesem Falle beispielsweise als Lösung von 500 ml in einem Beutel oder einer Flasche vor. Es kann jedoch auch auf ein Volumen von beispielsweise 250 ml portioniert sein, wobei sich dann die auf eine Tagesdosis bezogenen Mengenangaben auf ein Volumen von 500 ml beziehen.

Da zu Behandlungsbeginn eines im Sinne der Erfindung kritisch oder chronisch Kranken die für eine vollständige Ernährung erforderlichen Energie- und Stickstoffträger möglichst parenteral verabreicht werden, weil sie auf enteralem Wege nicht oder kaum toleriert werden, muss das zu Behandlungsbeginn enteral zu verabreichende Supplement I nahezu kalorienfrei sein, d.h. sollte eine Gesamtenergiemenge von 400 kcal pro Tag nicht überschreiten. Aus diesem Grunde sollte das erfindungsgemäße, für den Behandlungsbeginn eines kritisch oder chronisch Kranken vorgesehene enterale Supplement mit Ausnahme der Komponente c) fettfrei sein, und als Proteinbestandteile höchstens die Aminosäuren Glutamin, Glycin und ggf. Alanin (beispielsweise im Dipeptid zusammen mit Glutamin) und Cystein (als Antioxidans) aufweisen und sollte eine Menge von maximal 20 g Kohlenhydrate pro Tagesdosis (vorzugsweise in Form von Maltodextrinen) nicht überschreiten.

Beispiele zum Anwendungsbereich von Supplement I sind:
· Parenteral ernährte Patienten ohne Kontraindikation für die enterale Substratzufuhr bei gleichzeitig hochgradiger Intoleranz gegenüber herkömmlichen enteralen Nährlösungen (z.B. bei SIRS, Sepsis, hochdosierter Catecholamintherapie, Multiorganversagen, Verbrennungen),
· Große Abdominalchirurgie,
· Patienten, die vor diagnostischen Maßnahmen und vor operativen Eingriffen nüchtern bleiben müssen,
· Entzündliche Darmerkrankungen (M. Crohn, Colitis ulcerosa),
· Mucositis, Stomatitis (nach Chemo-, Radiotherapie, Knochenmarkstransplantationen),
· Kurzdarmsyndrom.
· akute Pankreatitis.

Die Erfindung betrifft auch die Verwendung der oben genannten Komponenten a) bis c) zur Herstellung eines enteral zu verabreichenden Supplements zur Behandlung der oben genannten Krankheiten.

### Supplement II:

Nach erkennbar eingetretener Stabilisierung der Darmfunktion, dann wenn größere Volumina des beispielsweise 500 ml umfassenden Supplements I toleriert werden, kann ein Teil der parenteralen Vollnahrung nun über das an diese Aufgaben adaptierte enterale Supplement II appliziert werden. In diesem Fall können dem enteralen Supplement zusätzlich zu den Nährstoffen, die bereits im kleinvolumigen Anfangssupplement (Supplement I) enthalten sind, bezogen auf eine Tagesdosis, bis zu maximal 30 g Protein, insbesondere in Form eines Proteinhydrolysats aus Molke, und/oder bis zu maximal 45 g Fett, insbesondere in Form mittelkettiger (C₆ - C₁₂) Triglyceride (MCT) und essentieller Fettsäuren, zugesetzt sein. Die Tagesdosis für die Kohlenhydrate (vorzugsweise in Form von Maltodextrinen) im größervolumigen enteralen Supplement II für einen Mangelernährten und einen kritisch oder chronisch Kranken kann dann, neben einer evtl. parenteralen Teilernährung, angehoben werden, beispielsweise bis auf 115 g.

Darüber hinaus ist es vorteilhaft, dem zusätzlich zur parenteralen Teiler-nährung enteral zu verabreichenden Supplement II bis zu maximal 1 mg Chrom, beispielsweise in der Form von Chromtrichlorid, zuzusetzen.

Ein derartig zusammengesetztes, einem Mangelernährten oder kritisch bzw. chronisch Kranken neben einer evtl. parenteralen Teilernährung oder neben einer teilweisen enteralen/oralen Ernährung zu verabreichendes Supplement, kann üblicherweise pro Tagesdosis ein Volumen bis zu 1000 ml einnehmen und verfügt dann über bis zu 1000 kcal. Dieses Supplement kann dann in einem Beutel oder einer Flasche auf Volumina von 1000 ml, 500 ml und 250 ml portioniert sein, wobei sich die für eine Tagesdosis angegebenen Mengenangaben auf ein Volumen von 1000 ml beziehen.

Beispiele zum Anwendungsbereich von Supplement II sind:
· Parenteral ernährte Patienten ohne Kontraindikation für die enterale Substratzufuhr bei weniger ausgeprägter Intoleranz gegenüber herkömmlichen enteralen Nährlösungen,
· Mangelernährung bei
   - chronischen Darmerkrankungen,
   - geriatrischen Patienten,
   - neurologischen Störungen,
   - Mukositis / Stomatitis,
   - Tumorkachexie,
   - Präventive präoperative Ernährung. Die Erfindung betrifft auch die Verwendung der oben genannten Komponenten a) bis c) zur Herstellung eines enteral zu verabreichenden Supplements zur Behandlung der oben genannten Krankheiten.

Die nachstehenden Mengenangaben für die jeweiligen definierten Substrate im erfindungsgemäßen Supplement, bezogen auf eine Tagesdosis, stellen für den Mangelemährten sowie für den kritisch oder chronisch Kranken eine ausge-wogene Mischung dar, wobei die durch katabole Belastungen induzierten Verluste ausgeglichen und damit therapiert oder aber die bereits zu Beginn der Erkrankung auftretenden Mangelzustände noch vor dem Zusammenbruch der intestinalen Darmbarriere im Sinne einer Protektion aufgefangen werden können. Zudem wird sichergestellt, dass die synergistische Wirkung der in Kombination angebotenen Substrate genutzt und die bei sonst alleiniger Verabreichung einzelner Substrate in extrem hoher Dosierung auftretenden Ungleichgewichte mit konsekutiver Stoffwechselstörung verhindert werden können.

Glutamin ist für den Gesunden eine nicht-essentielle Aminosäure und spielt als Zwischenstufe im Stickstoffmetabolismus eine zentrale Rolle. Glutamin dient als Donator des Stickstoffs bei der Synthese von Purinen, Pyrimidinen, Nucleotiden, Aminozuckern und Glutathion und stellt das wichtigste Substrat für die Bildung von Ammoniak in der Niere dar (Regulation des Säure-Base-Gleichgewichts). Glutamin dient ferner als Stickstoff-Transportsubstanz zwischen zahlreichen Geweben. Glutamin ist schließlich der wichtigste Energielieferant für den Stoffwechsel der Zellen des Gastrointestinaltraktes (Enterozyten, Kolonozyten) sowie für schnell proliferierende Zellen, wie die des Immunsystems.

Bei kritisch Kranken, z.B. Patienten nach einer Elektivoperation, nach größeren Traumen, Verbrennungen, Infektionen aber auch im Rahmen einer Pankreatitis, gehen die erhöhten katabolen und metabolen Zustände - unabhängig vom gerade herrschenden Ernährungszustand - mit einer signifikanten Herabsetzung der Glutaminkonzentration in Muskelzellen einher. Des weiteren werden periphere Glutaminspeicher während einer katabolen Stresssituation oder in tumortragenden Wirten schneller abgebaut, wobei die Aminosäure als Energiequelle bevorzugt zum Intestinum bzw. den Tumoren verfrachtet wird. Der beschleunigte Abbau führt dann dort zu einer Verarmung an Glutamin, was zur Folge hat, daß dann den Enterocyten und Immunocyten Glutamin fehlt. Daher wurde vorgeschlagen, Glutamin während kataboler Stresssituationen, wie einem Trauma oder einer Sepsis, als bedingt essentielle Aminosäure zu betrachten (vergl. EP-A-238,553).

Es ist daher vorteilhaft, bei der Behandlung gestresster und mangelernährter Patienten eine zusätzliche luminale (enterale) Ernährung des Darms mit Glutamin vorzunehmen; um die Funktion der Darmbarriere und den Immunstatus zu verbessern. Die Konzentration des Glutamins sollte dabei im Bereich von 15 - 70 g, vorzugsweise von 20 bis 50 g, insbesondere 20 bis 45 g, pro Tagesdosis liegen. Wird die Supplement-Lösung kurz vor ihrer beabsichtigten Verabreichung hergestellt, kann Glutamin in freier Form oder als Di- oder Tripeptid mit Ala und/oder Gly zugesetzt werden. Ist dagegen eine Aufbewahrung der fertigen Supplementlösung und/oder eine Sterilisation der fertigen Supplementlösung vorgesehen, ist Glutamin vorzugsweise in Form des Dipeptids mit Ala oder Gly einzusetzen. In diesem Falle bezieht sich die Mengenangabe auf den Gewichtsanteil des Glutamins im jeweiligen Dipeptid.

Es ist bekannt, daß Mangelernährte oder kritisch bzw. chronisch Kranke einem erhöhten oxidativen Stress, wie er z.B. besonders ausgeprägt nach einem lschämie/Reperfusionsschaden zu beobachten ist, ausgesetzt sind. Dabei kommt es zu einer Deregulation von Enzymsystemen, was einen erhöhten Anfall von toxischen Sauerstoffradikalen zur Folge hat. Zur Abwehr des oxidativen Stresses unterhält der Körper unter normalen Bedingungen ein Reservoir unterschiedlicher reduzierter Verbindungen (= Antioxidantien), wie z.B. Ascorbinsäure, Carotinoide, Dihydroliponsäure. Aber auch Spurenelemente, wie Selen und Zink, können als Antioxidantien wirken. Diese endogenen Antioxidantien reichen aber während einer Mangelernährung, sowie während chronischer Erkrankungen oder nach einem intensiven Stressereignis, dem kritisch Kranke ausgesetzt sein können, nicht aus, um die hohe Konzentration der gebildeten freien Radikale abzufangen oder schon in ihrer Entstehung zu verhindern. Dies bewirkt, dass sich im Rahmen einer generalisierten Entzündung das pathologische Erscheinungsbild einer systemischen Inflammation noch weiter steigert. Daher ist die Bereitstellung einer angemessenen Zufuhr an Antioxidantien dann angezeigt, wenn der Wirkung freier Radikale und dem daraus folgenden oxidativen Schaden entgegengewirkt werden soll.

Zudem ist bekannt, dass Antioxidantien während der Beseitigung der freien Radikale im Organismus hinsichtlich ihrer Regeneration in synergistischer Weise voneinander abhängig sind, indem sie eine Antioxidans-Spirale bilden. So werden z.B. Vitamin C, Vitamin E, Glutathion und NADP oxidiert und erlangen nach ihrer Reduktion wieder ihre Aktivität zurück. Eine derartige Antioxidans-Spirale ist auch für die Wechselwirkung der Vitamine C und E, Selen und Zink belegt.

Wegen dieser sich gegenseitig ergänzenden antioxidativen Wirkung ver-schiedener Substrate ist es daher sinnvoll, mindestens zwei der nachfolgend genannten Antioxidantien, vorzugsweise jedoch mehrere, insbesondere aber die Vitamine C und E, β-Carotin sowie die Spurenelemente Selen und Zink in Kombination miteinander dem erfindungsgemäßen Supplement neben den beiden anderen essentiellen Substraten (Glutamin und kurzkettige Fettsäuren bzw. deren Vorläufern) zuzusetzen.

Beispiele für Antioxidantien sind Vitamine mit Antioxidans-Eigenschaften, wie Vitamin C oder Vitamin E; Aminosäuren oder Aminosäure-Derivate mit Antioxidans-Eigenschaften, wie S-Adenosylmethionin, Cystein, Cystin oder Glutathion; Aminosulfonsäuren, wie Taurin; Spurenelemente mit Antioxidans-Eigenschaften, wie Selen oder Zink; Polyphenole und Carotinoide, bevorzugt β-Carotin.

Bei Mangelernährten, chronisch oder kritisch Kranken ist der Bedarf an Vitamin C größer als bei Gesunden. So wurde beobachtet, dass die Ascorbinsäurekonzentration im Plasma von Intensivpatienten trotz Gabe von über 200 mg Vitamin C pro Tag deutlich unter dem Normalwert von Gesunden lag. Bei Tieren mit Verbrennungen ließen sich durch frühe Gaben großer Mengen an Ascorbinsäure die für eine Notfalltherapie benötigten Flüssigkeitsmengen verringern. Da Vitamin C auch bei der Synthese des Collagens eine Rolle spielt, sind bei derartigen Krankheitszuständen hohe Gaben an Ascorbinsäure umso mehr von Vorteil, wenn es der Wundheilung bedarf. Daher können die hohen Vitamin C-Gaben im enteralen Supplement I und II als geeigneter Ausgleich für die sonst bei Mangelernährten, chronisch oder kritisch Kranken auftretenden Vitamin C-Mangelerscheinungen erachtet werden. Pro Tagesdosis kann im erfindungsgemäßen enteralen Supplement daher Vitamin C mit einer Menge im Bereich von 0,5 bis 4 g, vorzugsweise von 1 bis 2,5 g, enthalten sein.

Das lipidlösliche Vitamin E (Tocopherol) schützt Proteine, Nucleinsäuren und vor allem in Membranen eingebaute ungesättigte Fettsäuren vor der Oxidation durch Sauerstoffradikale. Kritisch oder chronisch Kranke zeigen sehr niedrige Vitamin E-Spiegel. Als wesentliche Ursache für Sekundärschäden nach traumatisch bedingten Schädelverletzungen des Menschen werden Schädigungen durch freie Radikale vermutet. Experimentell konnte gezeigt werden, dass frühe Zusätze von α-Tocopherol, oder von α-Tocopherol in Kombination mit Ascorbinsäure, den Gehalt von Lipidperoxidationsprodukten im Gehirn reduzieren können, wodurch eine Schutzwirkung gegen die Ausbreitung des Zellschadens ausgeübt wird. Das erfindungsgemäße enterale Supplement kann daher pro Tagesdosis einen Vitamin E-Gehalt von 0,2 bis 2 g, vorzugsweise von 0,3 bis 1,2 g, aufweisen.

In der Literatur gibt es viele Hinweise dafür, daß Carotinoide, darunter insbesondere β-Carotin, außer dessen bekannter Fähigkeit, angeregte Zustände von Triplett- und Singulettsauerstoff abzufangen, auch Peroxyl-Radikale und andere Formen von aktivem Sauerstoff abfangen können. Diese Eigenschaften sind insbesondere bei Reperfusionsschäden von Bedeutung. Es ist davon auszugehen, dass Patienten mit Ischämie/Reperfusions-schädigungen der Gewebe beträchtliche Mengen an Singulett-Sauerstoff produzieren, wobei dann β-Carotin das spezifische Mittel zur Neutralisation dieser besonders reaktiven Sauerstoffspezies darstellt. In einer Tagesdosis des erfindungsgemäßen enteralen Supplements I oder II kann daher β-Carotin in einer Menge von 5 - 80 mg, vorzugsweise von 10 bis 60 mg, enthalten sein.

Selen stellt als Strukturbestandteil des aktiven Zentrums der Glutathion-peroxidase einen essentiellen Bestandteil des Systems der intrazellulären Antioxidantien dar.

Dieses Selen-abhängige Enzym spielt die Hauptrolle beim Schutz der Zellen vor Peroxidationsschäden, insbesondere vor einer Lipidperoxidation. Selen scheint daher eine direkte Rolle bei der Regulation entzündlicher Prozesse zu spielen.

Bei systemischer Entzündungsreaktion oder Sepsis unterliegen Patienten einem starken oxidativen Stress. Bei diesen Patienten zeigen sich daher schnell stark erniedrigte Selenspiegel, welche mit zunehmenden renalen Verlusten sowie einer Reduktion der Peroxidaseaktivität und der Zunahme von Lipidperoxidationsprodukten einhergehen. Einige Patienten mit spezifischen Krankheitsbildern sind dabei besonders anfällig: Patienten mit Verbrennungen, Trauma, sowie Hämodialysepatienten. Bevorzugterweise ist daher für das enterale Supplement eine tägliche Selengabe von 0,2 bis 1 mg, vorzugsweise von 0,2 bis 0,6 mg, vorgesehen, um bei Mangelerkrankten sowie chronisch oder kritisch Kranken eine Normalisierung verschiedener biologischer Funktionen zu erreichen.

Zink stellt für mehr als 100 Enzyme einen essentiellen Cofaktor dar. Es konnte gezeigt werden, daß bei einem Zink-Mangel die Funktion der T-Helferzellen und die Aktivität der natürlichen Killerzellen beeinträchtigt wird. Darüber hinaus sind auch Behinderungen sowohl der antigenassoziierten und immun-regulatorischen Funktion der Makrophagen als auch der Interleukin-1-Freisetzung zu beobachten. Der Agressionsstoffwechsel von Mangelernährten sowie kritisch oder chronisch Kranken ist die Ursache für einen erhöhten Verlust von Zink mit dem Harn. Bei Patienten mit Verbrennungen kann auch ein Zinkmangel infolge von Verlusten über die Haut und bei Traumapatienten infolge einer gesteigerten Diurese auftreten. Je nach Art der Krankheit wird ein Zusatz von 15-40 mg Zink vorgeschlagen. Um den Immunstatus kritisch Kranker zu verbessern, ist daher vorzugsweise für das erfindungsgemäße enterale Supplement I und II eine tägliche Zinkdosis von 10 bis 60 mg, besonders bevorzugt von 20 bis 40 mg, vorgesehen.

Der Begriff der kurzkettigen Fettsäuren (SCFA) umfasst im einzelnen die Essigsäure, die Propionsäure die Buttersäure und die Pentansäure (C₂-C₅). Diese können durch mikrobielle Fermentation von Kohlenhydraten im Gastrointestinaltrakt von Säugern gebildet werden. Butyrat kommt auf natürlicher Basis in vielen Früchten und Gemüsen vor. Die ergiebigste Quelle ist Milchfett (Butter) (3-4% Butyrat), wo es als Ester des Glycerins vorkommen kann.

Acetat, Propionat und Butyrat werden durch die Darmbakterien im nahezu konstanten molaren Verhältnis von 60:25:15 gebildet und schnell von der intestinalen Mukosa absorbiert. Sie sind relativ kalorienreich, werden vom intestinalen Epithel und der Leber verstoffwechselt und stimulieren die Wasser- und Natriumabsorption im Dickdarm. Sie wirken auf die intestinale Mukosa trophisch. Zudem dienen sie als Nahrungsmittel und wirken als echte essentielle Substrate, da die Organfunktion geschwächt ist, wenn sie fehlen.

Präkursoren für die Bereitstellung von SCFA sind die Ballaststoffe. Daher läßt sich auf die Bildung von SCFA über eine Änderung der Ballaststoffgaben einwirken. Im Gegensatz dazu lässt sich über eine Entfernung der Ballaststoffe aus der Nahrung die Butyratkonzentration signifikant herabsetzen. Darüber hinaus konnte gezeigt werden, daß Butyrat für Kolonozyten das bevorzugte Energiesubstrat darstellt. Butyrat spielt auch eine Rolle bei der Verhinderung bestimmter Arten von Colitis. Beim experimentellen Kurzdarmsyndrom bewirkt ein Zusatz von Butyrat zur enteralen Nahrung einen beschleunigten Ausfluß von Aminosäuren aus der Pfortader, was darauf hinweist, daß Butyrat seine trophische Wirkung hauptsächlich im und über das Kolon ausübt. Es konnte auch gezeigt werden, daß nach oraler Verabreichung von Glycerintributyrat in Tagesdosen von 50 bis 400 mg/kg Körpergewicht über einen Zeitraum von 3 Wochen sich die Butyratkonzentration im Plasma bis auf nahezu 0,5 mMol anheben ließ, ohne dass toxische Nebenwirkungen nachweisbar waren (vergl. B.A.Conley et al. In Clinical Cancer Research, Vol. 4, 629-634, 1998).

Somit kann angenommen werden, dass kurzkettige Fettsäuren, wie Butyrat, als zusätzliches Substrat der enteralen Supplemente I und II zu einer Verbesserung der Digestion und Absorption im Darm beitragen können. Daher sind diese Verbindungen oder deren Derivate, wie Tributyrin, mit einer Tagesdosis von 0,5 bis 10 g, vorzugsweise von 1 bis 6 g, im erfindungsgemäßen Supplement enthalten.

Zur Unterstützung und Verbesserung des Immunstatus bei Mangelernährten sowie bei kritisch oder chronisch Kranken bedarf es einer gesteigerten Proliferation von Immunzellen, was eine erhöhte Syntheserate für DNA und RNA bedingt. Während den auf ein Trauma folgenden Infektionen besteht daher ein gesteigerter Bedarf an Nucleotiden, um der erforderlichen Produktion von Immunzellen gerecht zu werden. Die Synthese zu den Polynucleotidketten der DNA und RNA aus den entsprechenden energiereichen Desoxyribo-nucleosid- bzw. Ribonucleosidtriphosphaten wird von den jeweiligen DNA- bzw. RNA-Polymerasen katalysiert. Die energiereichen Desoxyribonucleosid- bzw. Ribonucleosidtriphosphate entstehen über Phosphorylierungsreaktionen aus den entsprechenden Desoxyribonucleosid- bzw. Ribonucleosidmono-phosphaten (= Nucleotide).

Nucleotide stellen also wichtige Komponenten für die Synthese von DNA und RNA dar. Eine bedarfsgerechte Nucleotidsynthese erfordert genügende Mengen an Purinen und Pyrimidinen. Beim Gesunden werden diese effektiv aus der Nahrung absorbiert, in der sie normalerweise in einer Menge von 1-2 g pro Tag enthalten sind. Purine und Pyrimidine werden entweder de-novo synthetisiert oder über Rückgewinnungsreaktionen im Nucleotidstoffwechsel gewonnen (Salvage-pathway), wodurch Intermediate des Nukleinsäureabbaus vor einem Totalabbau gerettet und für den erneuten Aufbau von Nukleinsäuren wiederverwendet werden können. Dadurch kann die Zelle große Mengen an Energie einsparen.

Bei hinreichender Proteinaufnahme stellt beim Gesunden die de-novo-Synthese die Hauptquelle zur Aufrechterhaltung der Nucleotidverfügbarkeit dar, wobei Glutamin der Hauptdonator für den Stickstoff ist.

Beim Mangelernährten und dem kritisch oder chronisch Kranken ist die ausreichende Verfügbarkeit von Nukleinsäuren möglicher Weise eingeschränkt, da während einer katabolen Belastung die Expression von Synthese-Enzymen im denovo-Syntheseweg offensichtlich beeinträchtigt ist, was zu einer Verarmung an Nucleotiden führen kann. Eine verminderte Verfügbarkeit von Nucleotiden hat sodann eine Beeinträchtigung der T-Zell-Funktion, eine Reduktion der Aktivität von natürlichen Killerzellen, verzögerte Wirts-Reaktionen, eine Unterdrückung der Proliferation von Lymphozyten sowie eine verminderte Interleukin-2-Produktion zur Folge. Es konnte gezeigt werden, daß beim kritisch Kranken eine Entfernung von Nucleotiden aus der Nahrung die Fähigkeit zur Phagozytose herabsetzt sowie die Clearance von experimentell zugeführten Pathogenen verschlechtert. Durch eine Wiederaufnahme von Nucleotiden in die Nahrungszufuhr gestalteten sich die meisten dieser Wirkungen reversibel.

In bevorzugter Ausführungsform werden dem erfindungsgemäßen enteralen Supplement daher zur Stützung und Verbesserung des Immunstatus Präkursoren für die Synthese von DNA, RNA und/oder energiereichen Phosphaten zugesetzt, insbesondere in Form von Nucleotiden, vorzugsweise aus Hefe. Eine Tagesdosis sollte bevorzugterweise im Bereich von 1,5 bis 15 g liegen, besonders bevorzugt im Bereich von 2 bis 6 g.

Weitere Zusätze zum erfindungsgemäßen enteralen Supplement können Ribose, Folsäure, B-Vitamine und Lysophosphatidsäure sein.

Glycin ist eine nicht-essentielle Aminosäure und gewöhnlich in allen Lösungen zur künstlichen Ernährung enthalten. Nach neueren Untersuchungen verfügt Glycin über zytoprotektive sowie antiinflammatorische und antineoplastische Eigenschaften. Es konnte zudem gezeigt werden, daß während einer Hypoxie (herabgesetzter O₂-Partialdruck) Glycin die Integrität der Zellmembranen aufrechterhält und dadurch eine Freisetzung intrazellulärer Enzyme verhindern kann. Nach Verabreichung von Lipopolysacchariden kann Glycin die Synthese und Freisetzung proinflammatorischer Cytokine und Eicosanoide in den Kupfer-Zellen der Leber hemmen, indem es den Ca-Spiegel im Cytosol senkt.

Wegen seiner Ca-antagonistischen Effekte, welche sich infolge der Hemmwirkung auf die Bildung proinflammatorischer Cytokine und Eicosanoide (2er- und 4er-Serie) vorteilhaft auf den Immunstatus auswirken, kann bevorzugterweise Glycin im erfindungsgemäßen Supplement in einer Menge von 5 - 70 g, besonders bevorzugt von 10 bis 40 g, bezogen auf eine Tagesdosis zugesetzt sein. Wird Glycin zusammen mit Glutamin als Dipeptid eingesetzt, bezieht sich die Mengenangabe auf den Gewichtsanteil des Glycins im Dipeptid.

In einer besonders bevorzugten Ausführungsform enthält das erfindungsgemäße Supplement, bezogen auf eine Tagesdosis: 20-45 g, insbesondere 30 g, Glutamin, 5-20 g, insbesondere 10 g, Glycin, 1,5-5 g, insbesondere 2 g, Nucleotide, 0,5-5 g, insbesondere 1 g, Tributyrin, 5-50 g Maltodextrine, 2-30 mg, insbesondere 10 mg, β-Carotin, 200-1000 mg, insbesondere 500 mg, Vitamin E, 500-2000 mg, insbesondere 1500 mg, Vitamin C, 200-600 µg, insbesondere 300 µg, Selen und 10-30 mg, insbesondere 20 mg, Zink.

Seit langem ist bekannt, dass Chrom mit einer Verbesserung der Glucose-Toleranz in Verbindung gebracht wird und dass bei Chrommangel die Verwertbarkeit von Glucose beeinträchtigt ist. Es konnte gezeigt werden, dass beim nicht-Insulin-abhängigen Diabetes mellitus die Nachfrage nach Insulin zurückging, was darauf hinweist, dass Chrom die Insulinwirkung zu steigern vermag.

Bei Mangelernährten und kritisch bzw. chronisch Kranken kann eine starke Insulin-Resistenz auftreten, so dass bei diesen Patienten sich die zusätzliche Gabe von Chrom als vorteilhafte therapeutische Maßnahme erwiesen hat. Es ließ sich auch zeigen, dass dreiwertiges Chrom untoxisch ist. So ergab eine Autopsie einer weiblichen Patientin, die 21 Jahre lang Chrom in parenteraler Form erhielt, keinen Hinweis auf karzinogene Eigenschaften.

Es ist daher vorteilhaft, dem zusätzlich zur parenteralen Teilernährung enteral zu verabreichenden Supplement bis zu maximal 1 mg Chrom zuzusetzen.

Weitere mögliche Bestandteile des erfindungsgemäßen Supplements sind Proteine, die mit bis zu 30 g, bezogen auf eine Tagesdosis, enthalten sein können und/oder zusätzliche (über den Gehalt an kurzkettigen Fettsäuren oder deren Vorläufern hinausgehende) Fette, die mit bis zu 45 g, bezogen auf eine Tagesdosis, enthalten sein können.

Proteine werden vorzugsweise in Form eines Proteinhydrolysats eingesetzt.

Zusätzliche Fette können mittelkettige und/oder langkettige Fettsäuren sein, die gesättigt und/oder ungesättigt sein können. Auch Derivate dieser Fettsäuren, wie Salze, Ester, insbesondere Glyzerinester oder Phospholipide, oder Amide können eingesetzt werden. Mittelkettige Fettsäuren weisen 6-12 Kohlenstoffatome auf. Langkettige Fettsäuren weisen 13 bis 22 Kohlenstoffatome auf.

Lipide können Aufgaben wahrnehmen, die über deren Funktion als Energieträger oder als zelluläre Strukturbestandteile weit hinausgehen. Lipide sind als pharmakologische Wirksubstanzen zu betrachten, welche durch Ernährung bereitgestellt werden. Dies trifft insbesondere für die mehrfach ungesättigten Fettsäuren (PUFAs) zu.

Mit der enteralen Aufnahme erhöhter Mengen an n-3 mehrfach ungesättigten Fettsäuren (n-3 PUFAs), wie sie z.B. in Fischöl als Eicosapentaensäure (EPA) und Docosahexaensäure (DHA) anzutreffen sind, verändert sich das Verhältnis von n-3 zu n-6-PUFAs im Phospholipidspektrum zugunsten der n-3 PUFAs. Dies hat Einfluß auf die Immunfunktion, wobei im wesentlichen zwei Auswirkungen zum Tragen kommen, welche zudem der gegenseitigen Beeinflussung unterliegen können:
1. Eine Änderung der Membranfluidität und
2. Eine Änderung der Freisetzung membrangebundener Lipide, welche durch Hydrolyse aus membranständigen Phospholipiden entstehen.

Ad 1) Änderungen der Fluidität können die Rezeptorbindung von Cytokinen sowie weiterer Agonisten beeinflussen. Der Zustand der Membranfluidität hat zudem Einfluß auf die intrazelluläre Signalübertragung, was sich beispielsweise auf die Aktivität von G-Proteinen auswirken und konsekutiv zur Aktivitätsänderung von Enzymsystemen führen kann (Adenylat-Kinase, Phospholipase A₂, Phospholipase C). Daneben sind mehrfach ungesättigte Fettsäuren wichtige Präkursoren zur Bildung sogenannter "second messenger" wie Diacylglycerin und Ceramide. Die Applikation von Fisch-Öl ist daher mit profunden Änderungen der intrazellulären Signaltransduktion verbunden, welche Einfluß auf die Produktion und Freisetzung von Cytokinen, Interleukinen sowie Interferonen nehmen können.

Ad 2) Unter dem Einfluß des Enzyms Phospholipase A₂ können PUFAs aus Membran-Phospholipiden freigesetzt werden. Nach ihrer Hydrolyse werden sie als Präkursoren zur Bildung von Lipidmediatoren herangezogen. Im Cyclooxygenase- und Lipoxygenaseweg werden mehrfach ungesättigte Fettsäuren zu Eicosanoiden umgewandelt. Die wichtigsten Vorteile der sich aus n-3 PUFAs ableitenden Eicosanoide können im Vergleich zu den sich aus n-6 PUFAs ableitenden Eicosanoiden wie folgt zusammengefasst werden:
1. Das im Cyclooxygenaseweg aus Eicosapentaensäure (EPA) abgeleitete Thromboxan A₃ (TxA₃) hat eine geringer aggregierende und damit prothrombotische Wirkung auf Thrombocyten als TxA₂, das sich aus n-6 PUFAs ableitet.
2. EPA wird im Lipoxygenaseweg zu LTB₅ umgewandelt, welches eine wesentlich geringere Aktivität als LTB₄ (aus n-6 PUFAs) aufweist und daher eine deutlich verminderte chemotaktisch induzierte Migration sowie eine geringere endotheliale Adherenz immunkompetenter Zellen induziert.
3. Das sich von n-3 PUFAs ableitende PGE₂ hat eine deutlich geringere immunsupprimierende Wirkung als PGE₂, welches sich aus n-6 PUFAs ableitet.

Über diese Wirkmechanismen beeinflussen n-3 PUFAs die während eines Traumas oder einer Infektion beeinträchtigte Immunantwort in zweifacher Weise:
1. Es kommt zu einer Verminderung der häufig bestehenden Hyperinflammation.
2. Unter den Bedingungen des Stressstoffwechsels kommt es zu einer Stärkung der immunologischen Abwehrfunktionen.

Ad 1) Es konnte gezeigt werden, daß hyperinflammatorische Prozesse durch erhöhte Verabreichung von n-3-Fettsäuren gegenüber n-6-Fettsäuren abgeschwächt werden konnten. Insbesondere verminderte EPA die Freisetzung proinflammatorisch wirksamer Cytokine wie IL-1, IL-6 und TNF-α sowie -β. Zudem führte die Gabe von Fischöl zu einer reduzierten Freisetzung proinflammatorischer Substanzen wie Leukotrien B₄ und PAF sowie zu einer verminderten lokalen Bildung von Thromboxan A₂. Insbesondere am Beispiel des Morbus Crohn und der Colitis ulcerosa konnte nachgewiesen werden, dass durch die diätetische Zufuhr von Fischöl bestehende Zeichen der Inflammation gemildert werden können.

Ad 2) Bei kritisch Kranken gibt es Hinweise zum augmentierenden Einfluß von n-3 PUFAs auf die zelluläre Abwehrfunktion, welche die Reduktion von Prostaglandinen der 2er-Serie (z.B. PGE₂) mit einer verminderten Feedback-Hemmung und einern damit einhergehenden "Boostereffekt" auf die zelluläre Abwehrfunktion in Verbindung bringen. Diese Vorstellung wird von Befunden gestützt, die zeigen, daß die Gabe von n-3 PUFAs während SIRS oder Sepsis die Cytokinproduktion steigert, die Antigenpräsentation optimieren, die Splenozyten-Proliferation verstärken, die Fähigkeit zur Opsonisierung verbessern und die Mortalität reduziert.

Die Vorteile der enteralen Verabreichung von n-3 PUFAs können wie folgt zusammengefaßt werden:
1. Reduktion der inflammatorischen Immunantwort.
2. Wiederherstellung der Abwehrfunktion immunkompetenter Zellen durch Verminderung der Stress induzierten PGE₂-Freisetzung.
3. Antiarrhythmische Eigenschaften.
4. Antithrombotische Eigenschaften.
5. Erhaltung der Mikrozirkulation.

Aus diesem Grunde besteht der Fettanteil (maximal 45g Fett) im Supplement II neben mittelkettigen Triglyceriden (MCT) vorzugsweise aus Fischöl, das bekanntlich reich an den n-3-Fettsäuren EPA und DHA ist. Die n-3-Fettsäuren liegen dabei im Triglyceridverbund vor. Vorzugsweise kann das Supplement II dann einen Gehalt an n-3-Fettsäuren im Bereich von 5 bis 15 g, besonders bevorzugt im Bereich von 6 bis 10 g, bezogen auf eine Tagesdosis, aufweisen. Vorzugsweise soll(en) (ein) Fischöl(e) entweder einzeln oder in einem derartigen Verhältnis untereinander vermischt eingesetzt werden, dass das Verhältnis von n-3-Fettsäuren zu n-6-Fettsäuren etwa 1:1 beträgt.

In einer ganz besonders bevorzugten Ausführungsform enthält das erfindungsgemäße Supplement, bezogen auf eine Tagesdosis: 20-45 g, insbesondere 30 g, Glutamin, 5-20 g, insbesondere 10 g, Glycin, 1,5-5 g, insbesondere 2 g, Nucleotide, 0,5-5 g, insbesondere 1 g, Tributyrin, 5-100 g, insbesondere 90 g, Maltodextrine, 2-30 mg, insbesondere 10m g, β-Carotin, 200-1000 mg, insbesondere 500 mg, Vitamin E, 500-2000 mg, insbesondere 1500 mg, Vitamin C, 200-600 µg, insbesondere 300 µg, Selen, 10-30 mg, insbesondere 20 mg, Zink, 200-600 µg, insbesondere 400 µg, Chrom, 15-30 g, insbesondere 20 g, Proteinhydrolysat, insgesamt 15-30 g, insbesondere 22 g, zusätzliche Fette, davon 7-12 g, insbesondere 10 g, mittelkettige Fettsäuren und 4-8 g, insbesondere 6 g, n-3-Fettsäuren aus Fischöl mit einem n-3/n-6-Verhältnis von >= 1:1, weitere Vitamine, Spurenelemente und Mineralstoffe.

Die Erfindung wird anhand der folgenden Beispiele näher erläutert.

### Beispiel 1 (entsprechend Supplement I):

Ein einem Mangelernährten oder kritisch bzw. chronisch Kranken zusätzlich zu einer parenteralen Vollnahrung oder zusätzlich zu enteraler/oraler Ernährung enteral zu verabreichendes flüssiges Supplement für den Behandlungsbeginn wies, bezogen auf eine Tagesdosis, die folgende Zusammensetzung auf:

| Volumen: 500 ml | |
|---|---|
| Glutamin | 30 g |
| Glycin | 10 g |
| Maltodextrine | 16 g |
| Tributyrin | 1 g |
| Nucleotide | 2g |
| Antioxidantien: | |
| β-Carotin | 10 mg |
| Vitamin C | 1500 mg |
| Vitamin E | 500 mg |
| Selen | 300 µg |
| Zink | 20 mg |

Das Supplement verfügte über ca. 250 kcal und wies eine kalorische Dichte von ca. 0,5 kcal/ml, eine Osmolalität von 500 mosmol/kg Wasser und eine Osmolarität von 455 mosmol/Liter Wasser auf.

### Beispiel 2 (entsprechend Supplement II):

Ein einem Mangelernährten oder kritisch bzw. chronisch Kranken zusätzlich zu einer parenteralen Teilernährung oder zusätzlich zu enteraler/oraler Ernährung enteral zu verabreichendes flüssiges Supplement wies, bezogen auf eine Tagesdosis, die folgende Zusammensetzung auf:

| Volumen: 1000 ml | |
|---|---|
| Proteinhydrolysat (aus Molke) | 20 g |
| Glutamin | 30 g |
| Glycin | 10 g |
| Maltodextrine | 90 g |
| Tributyrin | 1 g |
| Nucleotide | 2 g |
| Antioxidantien: | |
| β-Carotin | 10 mg |
| Vitamin C | 1500 mg |
| Vitamin E | 500 mg |
| Selen | 300 µg |
| Zink | 20 mg |
| Fette: | 22 g |
| n-3-Fettsäuren (aus Fischöl) | 6 g |
| n-3/n-6-Verhältnis | 1/1 |
| Chrom | 400 µg |

Das Supplement verfügte über ca. 800 kcal und wies eine kalorische Dichte von ca. 0,8 kcal/ml, eine Osmolalität von 530 mosmol/kg Wasser und eine Osmolarität von 425 mosmol/Liter Wasser auf. Das gewichtsprozentuale Verhältnis Protein: Kohlenhydrate: Fett betrug näherungsweise 30 : 45 : 25. Das Supplement II enthält darüber hinaus weitere Vitamine und Spurenelemente sowie Mineralstoffe in Mengen, die mit dem Energiegehalt korrelierten.

Beispiel 3: Dieses Beispiel beschreibt den Einfluss der Verabreichung der erfindungsgemäßen Zusammensetzung auf den Plasmaspiegel von Operationspatienten

Chirurgischen Patienten wurden ab dem ersten Tag postoperativ über 10 Tage hinweg täglich je 1500 ml der nachfolgend beschriebenen Zusammensetzung kontinuierlich mittels Ernährungspumpe appliziert.

Die Tagesdosis der Zusammensetzung umfasste 30 g Glutamin, 10 g Glycin, 224 g Maltodextrine, 1 g Tributyrin, 10 mg β-Carotin, 1500 mg Vitamin C, 500 mg Vitamin E, 300 µg Selen, 20 mg Zink, 68 g Molkeprotein-Hydrolysat und 65 g Fette, davon 6g n-3-Fettsäuren aus Fischöl.

Den Patienten wurden vor der Operation, am ersten Tag nach der Operation (vor Beginn der Supplementation mit der Testlösung), sowie am fünften und elften Tag nach der Operation Blutproben entnommen. Anschließend wurde der Gehalt von ausgewählten Substraten im Plasma bestimmt.

Die nachfolgende Tabelle zeigt den Verlauf der ermittelten Plasmaspiegel an Glutamin, Zink, Selen, β-Carotin, Vitamin C und α-Tocopherol am Tag vor der Operation bzw. am ersten, fünften und elften Tag nach der Operation (Pre-OP, POD1, POD5 bzw. POD11).

**Tabelle**

| Plasmabestandteil | Glutamin (µmol/l) | Zink (µmol/l) | Selen (µg/l) | β-Carotin (µmol/l) | Vitamin C (µmol/l) | α-Tocopherol (µmol/l) |
|---|---|---|---|---|---|---|
| Pre-OP (n=14)^{*)} | 599±121 | 8,44±2,29 | 60,4±18,6 | 0,16±0,11 | 15,08±12,34 | 15,50±3,99 |
| POD1 (n=14)^{*)} | 508±165 | 4,38±1,57 | 46,0±20,1 | 0,12±0,08 | 10,06±8,54 | 11,08±2,76 |
| POD5 (n=13)^{*)} | 600±102 | 7,01±1,98 | 69,5±17,2 | 0,22±0,11 | 34,75±19,47 | 29,86±10,64 |
| POD11 (n=9)^{*)} | 597±83 | 9,20±3,16 | 104,2±35,8 | 0,47±0,22 | 34,51±13,37 | 40,20±14,94 |

| | | | | | | |
|---|---|---|---|---|---|---|
| ^{*)} Anzahl der untersuchten Patienten | | | | | | |

Die Ergebnisse zeigen, dass in allen Fällen zumindest eine Normalisierung der Plasmaspiegel erreicht und bei einigen Schlüsselsubstraten sogar eine deutliche Steigerung bzw. Verbesserung gegenüber der Ausgangssituation festgestellt werden konnte. Die Ergebnisse zeigen ferner, dass die Schlüsselsubstrate vom Menschen im Darm aufgenommen werden und somit zur Verbesserung des antioxidativen Status der Patienten beitragen.

## Patentansprüche

1. Enteral zu verabreichendes Supplement zur Erhaltung oder Wiederherstellung der Darmbarriere von kritisch oder chronisch Kranken sowie Mangelemährten, **dadurch gekennzeichnet,**
**dass** es einen Gesamtenerglegehalt pro Tagesdosis von bis zu 4185 kJ (1.000 kcal) aufweist und als Lösung, jeweils bezogen auf eine Tagesdosis, enthält:
a) Glutamin und/oder Glutamin-Vorläufer ausgewählt aus der Gruppe bestehend aus Glutamin-Estern, Glutamin-Amiden, N-alkylierten Glutaminen, Glutamin-Salzen, Keto-Vorläufern des Glutamins oder kurzkettigen, glutaminhaltigen Peptiden in einer Menge im Bereich von 15 bis 70 g, wobei sich die Mengenangaben bei Glutamin-Vorläufern auf deren Glutamin-Anteil beziehen.
b) mindestens zwei Vertreter aus der Gruppe der als Antioxidantien wirkenden Substanzen ausgewählt aus der Gruppe bestehend aus Vitaminen, Aminosäuren, Aminosäurederivaten, Aminosulfonsäuren, Spurenelementen, Polyphenolen und Carotinoiden, und
c) kurzkettige Fettsäuren mit zwei bis fünf Kohlenstoffatomen und/oder Salze oder Ester als Vorläufer kurzkettiger Fettsäuren mit zwei bis fünf Kohlenstoffatomen In einer Menge von 0,5 bis 10 g, wobei sich die Mengenangaben bei Vorläufern kurzkettiger Fettsäuren auf deren Anteil an kurzkettigen Fettsäuren beziehen.

2. Supplement nach Anspruch 1, **dadurch gekennzeichnet, dass** es eine Tagesdosis von 200 bis 1000 ml, bevorzugt von 500 ml, umfasst.

3. Supplement nach Anspruch 1, **dadurch gekennzeichnet, dass** es einen Gesamtenergiegehalt pro Tagesdosis von bis zu 1674 kJ (400 kcal) und eine Tagesdosis von bis zu 500 ml aufweist.

4. Supplement nach einem der Ansprüche 1 oder 2, **dadurch gekennzeichnet, dass** es sterilisiert und in gebrauchsfertiger flüssiger Form vorliegt.

5. Supplement nach einem der Ansprüche 1 bis 4, **dadurch gekennzeichnet, dass** es Glutamin und/oder Glutamin-Vorläufer, bevorzugt in Form von kurzkettigen, glutaminhaltigen Peptiden, besonders bevorzugt in Form von Dipeptiden, im Bereich von 20-45 g, berechnet als Glutamin, bezogen auf eine Tagesdosis, enthält.

6. Supplement nach einem der Ansprüche 1 bis 5, **dadurch gekennzeichnet, dass** es Vorläufer von kurzkettigen Fettsäuren in der Form von Glycerinestern kurzkettiger Fettsäuren, besonders bevorzugt Tributyrin, enthält.

7. Supplement nach einem der Ansprüche 1 bis 6, **dadurch gekennzeichnet, dass** die Antioxidantien ausgesucht sind aus der Gruppe Vitamin C, Vitamin E, S-Adenosylmethionin, Cystein, Cystin, Glutathion, Taurin, Selen, Zink, Polyphenole und Carotinoide, bevorzugt β-Carotin.

8. Supplement nach Anspruch 7, **dadurch gekennzeichnet, dass** es die Antioxidantien Vitamin C mit einem Gehalt im Bereich von 0,5 bis 4 g und/oder Vitamin E mit einem Gehalt im Bereich von 0,2 bis 2 g und/oder β-Carotin mit einem Gehalt im Bereich von 5 bis 80 mg und/oder Selen Mit einem Gehalt im Bereich von 0,2 bis 1 mg, vorzugsweise 0,3 bis 0,6 mg, und/oder Zink mit einem Gehalt im Bereich von 10 bis 60 mg, Jeweils bezogen auf eine Tagesdosis, enthält.

9. Supplement nach Anspruch 7, **dadurch gekennzeichnet**, es in Kombination die Antioxidantien Vitamin C, Vitamin E, β-Carotin, Selen und Zink enthält.

10. Supplement nach einem der Ansprüche 1 bis 9, **dadurch gekennzeichnet, dass** es zusätzlich Präkursoren für die Synthese von DNA, RNA und energlereichen Phosphaten enthält.

11. Supplement nach Anspruch 10, **dadurch gekennzeichnet, dass** die Präkursoren die entsprechenden Nucleotide sind.

12. Supplement nach Anspruch 11, **dadurch gekennzeichnet, dass** es die Nucleotide mit einem Gehalt im Bereich von 1,5 bis 15 g, bezogen auf eine Tagesdosis, enthält.

13. Supplement nach einem der Ansprüche 1 bis 12, **dadurch gekennzeichnet, dass** es zusätzlich eine oder mehrere Substanzen enthält aus der Gruppe Ribose, Folsäure, B-Vitamine und Lysophosphatidsäure.

14. Supplement nach Anspruch 1, **dadurch gekennzeichnet, dass** es Glycin als Substanz mit Ca-antagonistischem Effekt enthält.

15. Supplement nach Anspruch 14, **dadurch gekennzeichnet, dass** es Glycin mit einem Gehalt im Bereich von 5 bis 70 g, bezogen auf eine Tagesdosis, enthält.

16. Supplement nach einem der Ansprüche 1 bis 15, **dadurch gekennzeichnet, dass** es, bezogen auf eine Tagesdosis enthält: 20-45 g Glutamin, 5-20 g Glycin, 1,5-5 g Nucleotide, 0,5-5 g Tributyrin, 5-50 g Maltodextrine, 2-30 mg β-Carotin, 200-1000 mg Vitamin E, 500-2500 mg Vitamin C, 200-600 µg Selen und 10-30 mg Zink.

17. Supplement nach einem der Ansprüche 1 bis 16, **dadurch gekennzeichnet, dass** es in Kombination mit einer parenteral zu verabreichenden Vollnahrung als künstliches Ernährungssystem für einen Mangelernährten, kritisch oder chronisch Kranken dient.

18. Supplement nach einem der Ansprüche 1 bis 17, **dadurch gekennzeichnet, dass** es, bezogen auf eine Tagesdosis, zusätzlich Proteine mit einem bis zu 30 g reichenden Gehalt und/oder Fette mit einem bis zu 45 g reichenden Gehalt und/oder Chrom mit einem bis zu 1 mg reichenden Gehalt umfasst.

19. Supplement nach Anspruch 18, **dadurch gekennzeichnet, dass** es die zusätzlichen Proteine in Form eines Protoinhydrolysats enthält.

20. Supplement nach Anspruch 18 oder 19, **dadurch gekennzeichnet, dass** die zusätzlichen Fette mittelkettige Fettsäuren umfassen.

21. Supplement nach einem der Ansprüche 18 bis 20, **dadurch gekennzeichnet, dass** die zusätzlichen Fette einen hohen Gehalt an n-3-Fettsäuren umfassen.

22. Supplement nach Anspruch 21, **dadurch gekennzeichnet, dass** die n-3-Fettsäuren Eicosapentaensäure und/oder Docosahexaensäure umfassen.

23. Supplement nach Anspruch 21 oder 22, **dadurch gekennzeichnet, dass** das Fett ein Fischöl oder eine Mischung verschiedener Fischöle umfasst.

24. Supplement nach einem der Ansprüche 18 bis 23, **dadurch gekennzeichnet, dass** es, bezogen auf eine Tagesdosis, enthält: 20-45 g Glutamin, 5-20 g Glycin, 1,5-5 g Nucleotide, 0,5-5 g Tributyrin, 5-100 g Maltodextrine, 2-30 mg β-Carotin, 200-1000 mg Vitamin E, 500-2000 mg Vitamin C, 200-600 µg Selen, 10-30 mg Zink, 200-600 µg Chrom, 15-30 g Proteinhydrolysat, insgesamt 15-30 g zusätzliche Fette, davon 7-12 g mittelkettige Fettsäuren und 4-8 g n-3-Fettsäuren aus Fischöl mit einem n-3/n-6-Verhältnis von >= 1:1, weitere Vitamine, Spurenelemente und Mineralstoffe.

25. Verwendung der Komponenten a) bis c) gemäß Anspruch 1 zur Herstellung eines enteral zu verabreichenden Supplements zur Behandlung von parenteral ernährten Patienten ohne Kontraindikation für die enterale Substratzufuhr bei gleichzeitig hochgradiger Intoleranz gegenüber herkömmlichen enteralen Nährlösungen oder bei weniger ausgeprägter Intoleranz gegenüber herkömmlichen enteralen Nährlösungen, zur Behandlung bei der Mangelernährung oder bei der , großen Abdominalchirurgle, zur Behandlung von Patienten, die vor diagnostischen Maßnahmen und vor operativen Eingriffen nüchtern bleiben müssen, zur Behandlung von entzündlichen Darmerkrankungen, Mucositis, Stomatitis, bei Kurzdarmsyndrom und bei akuter Pankreatis.

## Claims

1. Supplement to be administered enterally to maintain or restore the intestinal barrier of the critically or chronically ill and people with malnutrition, **characterized in that** it has a total energy content per daily dose of up to 4185 kJ (1000 kcal) and comprises as solution, in each case based on a daily dose:
a) glutamine and/or glutamine precursor selected from the group consisting of glutamine esters, glutamine amides, N-alkylated glutamines, glutamine salts, keto precursors of glutamine or short-chain, glutamine-containing peptides in an amount in the range from 15 to 70 g, where in the case of glutamine precursors the stated amounts are based on the glutamine content thereof,
b) at least two representatives from the group of substances having antioxidant activity selected from the group consisting of vitamins, amino acids, amino acid derivatives, amino sulphonic acid, trace elements, polyphenols and carotenoids, and
c) short-chain fatty acids having two to five carbon atoms and/or salts or esters as precursors of short-chain fatty acids having two to five carbon atoms in an amount of from 0.5 to 10 g, where in the case of precursors of short-chain fatty acids the stated amounts are based on the short-chain fatty acid content thereof.

2. Supplement according to Claim 1, **characterized in that** it comprises a daily dose of from 200 to 1000 ml, preferably of 500 ml.

3. Supplement according to Claim 1, **characterized in that** it has a total energy content per daily dose of up to 1674 kJ (400 kcal) and a daily dose of up to 500 ml.

4. Supplement according to either of Claims 1 or 2, **characterized in that** it is in sterilized and liquid form ready for use.

5. Supplement according to any of Claims 1 to 4, **characterized in that** it comprises glutamine and/or glutamine precursors, preferably in the form of short-chain, glutamine-containing peptides, particularly preferably in the form of dipeptides, in the range 20-45 g, calculated as glutamine, based on a daily dose.

6. Supplement according to any of Claims 1 to 5, **characterized in that** it comprises precursors of short-chain fatty acids in the form of glycerol esters of short-chain fatty acids, particularly preferably tributyrin.

7. Supplement according to any of Claims 1 to 6, **characterized in that** the antioxidants are selected from the group of vitamin C, vitamin E, S-adenosylmethionine, cysteine, cystine, glutathione, taurine, selenium, zinc, polyphenols and carotenoids, preferably β-carotene.

8. Supplement according to Claim 7, **characterized in that** it comprises the antioxidants vitamin C with a content in the range from 0.5 to 4 g and/or vitamin E with a content in the range from 0.2 to 2 g and/or β-carotene with a content in the range from 5 to 80 mg and/or selenium with a content in the range from 0.2 to 1 mg, preferably 0.3 to 0.6 mg, and/or zinc with a content in the range from 10 to 60 mg, in each case based on a daily dose.

9. Supplement according to Claim 7, **characterized in that** it comprises the antioxidants vitamin C, vitamin E, β-carotene, selenium and zinc in combination.

10. Supplement according to any of Claims 1 to 9, **characterized in that** it additionally comprises precursors for the synthesis of DNA, RNA and energy-rich phosphates.

11. Supplement according to Claim 10, **characterized in that** the precursors are the corresponding nucleotides.

12. Supplement according to Claim 11, **characterized in that** it comprises the nucleotides with a content in the range from 1.5 to 15 g, based on a daily dose.

13. Supplement according to any of Claims 1 to 12, **characterized in that** it additionally comprises one or more substances from the group of ribose, folic acid, B vitamins and lysophosphatidic acid.

14. Supplement according to Claim 1, **characterized in that** it comprises glycine as substance having a Ca-antagonistic effect.

15. Supplement according to Claim 14, **characterized in that** it comprises glycine with a content in the range from 5 to 70 g, based on a daily dose.

16. Supplement according to any of Claims 1 to 15, **characterized in that** it comprises, based on a daily dose: 20-45 g of glutamine, 5-20 g of glycine, 1.5-5 g of nucleotides, 0.5-5 g of tributyrin, 5-50 g of maltodextrins, 2-30 mg of β-carotene, 200-1000 mg of vitamin E, 500-2500 mg of vitamin C, 200-600 µg of selenium and 10-30 mg of zinc.

17. Supplement according to any of Claims 1 to 16, **characterized in that** it is used in combination with a complete food to be administered parenterally as artificial nutrition system for a person with malnutrition or a critically or chronically ill patient.

18. Supplement according to any of Claims 1 to 17, **characterized in that** it comprises, based on a daily dose, in addition proteins with a content extending up to 30 g and/or fats with a content extending up to 45 g and/or chromium with a content extending up to 1 mg.

19. Supplement according to Claim 18, **characterized in that** it comprises the additional proteins in the form of a protein hydrolysate.

20. Supplement according to Claim 18 or 19, **characterized in that** the additional fats comprise medium-chain fatty acids.

21. Supplement according to any of Claims 18 to 20, **characterized in that** the additional fats comprise a high content of n-3 fatty acids.

22. Supplement according to Claim 21, **characterized in that** the n-3 fatty acids comprise eicosapentaenoic acid and/or docosahexaenoic acid.

23. Supplement according to Claim 21 or 22, **characterized in that** the fat comprises a fish oil or a mixture of different fish oils.

24. Supplement according to any of Claims 18 to 23, **characterized in that** it comprises, based on a daily dose: 20-45 g of glutamine, 5-20 g of glycine, 1.5-5 g of nucleotides, 0.5-5 g of tributyrin, 5-100 g of maltodextrins, 2-30 mg of β-carotene, 200-1000 mg of vitamin E, 500-2000 mg of vitamin C, 200-600 µg of selenium, 10-30 mg of zinc, 200-600 µg of chromium, 15-30 g of protein hydrolysate, a total of 15-30 g of additional fats, of which 7-12 g are medium-chain fatty acids and 4-8 g are n-3 fatty acids from fish oil with an n-3/n-6 ratio of >= 1:1, further vitamins, trace elements and minerals.

25. The use of components a) to c) as set forth in Claim 1 for producing a supplement to be administered enterally for the treatment of parenterally fed patients without contraindication for enteral supply of substrates with, at the same time, a high degree of intolerance to conventional enteral nutrient solutions or with less pronounced intolerance to conventional enteral nutrient solutions, for treatment in cases of malnutrition or major abdominal surgery, for the treatment of patients who must be fasted before diagnostic procedures and before surgical interventions, for the treatment of inflammatory bowel disorders, mucositis, stomatitis, in short-bowel syndrome and in acute pancreatitis.

## Revendications

1. Supplément à administrer par voie entérale pour entretenir ou rétablir la barrière intestinale chez des malades critiques ou chroniques ainsi que chez des sujets carencés, **caractérisé en ce qu'**il présente une teneur énergétique totale par dose journalière allant jusqu'à 4185 kJ (1 000 kcal) et qu'il contient, sous forme de solution, à chaque fois par rapport à une dose journalière :
a) de la glutamine et/ou un précurseur de la glutamine choisi dans le groupe formé par les esters glutamiques, les amides glutamiques, les glutamines N-alkylées, les sels glutamiques, les précurseurs cétoniques de la glutamine ou des peptides à chaîne courte contenant de la glutamine, à raison de 15 g à 70 g, les indications quantitatives pour les précurseurs de la glutamine se rapportant à leur teneur en glutamine,
b) au moins deux représentants du groupe des substances agissant en tant qu'anti-oxydants choisies dans le groupe formé par les vitamines, les acides aminés, les dérivés d'acides aminés, les acides aminosulfoniques, les oligo-éléments, les polyphénols,et les caroténoïdes, et
c) des acides gras à chaîne courte possédant deux à cinq atomes de carbone et/ou des sels ou des esters en tant que précurseurs d'acides gras à chaîne courte, à raison de 0,5 g à 10 g, les indications quantitatives pour les précurseurs d'acides gras à chaîne courte se rapportant à leur teneur èn acides gras à chaîne courte.

2. Supplément selon la revendication 1, **caractérisé en ce qu'**il comprend une dose journalière allant de 200 mL à 1 000 mL, de préférence de 500 mL.

3. Supplément selon là revendication 1, **caractérisé en ce qu'**il présente une teneur énergétique totale par dose journalière allant jusqu'à 1 674 kJ (400 kcal) et une dose journalière allant jusqu'à 500 mL.

4. Supplément selon l'une des revendications 1 et 2, **caractérisé en ce qu'**il se présente sous forme stérilisée et liquide prête à l'emploi.

5. Supplément selon l'une quelconque des revendications 1 à 4, **caractérisé en ce qu'**il contient de la glutamine et/ou des précurseurs de la glutamine, de préférence sous forme de peptides à chaîne courte contenant de la glutamine, de manière plus particulièrement préférée sous forme de dipeptides, à raison de 20 g à 45 g, exprimé en tant que glutamine, par rapport à une dose journalière.

6. Supplément selon l'une quelconque des revendications 1 à 5, **caractérisé en ce qu'**il contient des précurseurs d'acides gras à chaîne courte sous forme d'esters glycériques d'acides gras à chaîne courte, de manière plus particulièrement préférée, sous forme de tributyrine.

7. Supplément selon l'une quelconque des revendications 1 à 6, **caractérisé en ce que** les anti-oxydants sont choisis dans le groupe formé par la vitamine C, la vitamine E, la S-adénosylméthionine, la cystéine, la cystine, le glutathion, la taurine, le sélénium, le zinc, les polyphénols et les caroténoïdes, de préférence le β-carotène.

8. Supplément selon la revendication 7, **caractérisé en ce qu'**il contient les anti-oxydants vitamine C à raison de 0,5 g à 4 g et/ou vitamine E à raison de 0,2 mg à 2 g et/ou β-carotène à raison de 5 mg à 80 mg et/ou sélénium à raison de 0,2 mg à 1 mg, de préférence de 0,3 mg à 0,6 mg, et/ou zinc à raison de 10 mg à 60 mg, à chaque fois par rapport à une dose journalière.

9. Supplément selon la revendication 7, **caractérisé en ce qu'**il contient, de manière combinée, les anti-oxydants vitamine C, vitamine E, β-carotène, sélénium et zinc.

10. Supplément selon l'une quelconque des revendications 1 à 9, **caractérisé en ce qu'**il contient en plus des précurseurs pour la synthèse de l'ADN, de l'ARN et de phosphates riches en énergie.

11. Supplément selon la revendication 10, **caractérisé en ce que** les précurseurs sont les nucléotides correspondants.

12. Supplément selon la revendication 11, **caractérisé en ce qu'**il contient les nucléotides à raison de 1,5 g à 15 g, par rapport à une dose journalière.

13. Supplément selon l'une quelconque des revendications 1 à 12, **caractérisé en ce qu'**il contient en plus une ou plusieurs substances du groupe formé par le ribose, l'acide folique, les vitamines B et l'acide lysophosphatidique.

14. Supplément selon la revendication 1, **caractérisé en ce qu'**il contient de la glycine en tant que substance ayant un effet antagoniste du Ca.

15. Supplément selon la revendication 14, **caractérisé en ce qu'**il contient de la glycine à raison de 5 g à 70 g par rapport à une dose journalière.

16. Supplément selon l'une quelconque des revendications 1 à 15, **caractérisé en ce qu'**il contient, par rapport à une dose journalière : 20 g à 45 g de glutamine, 5 g à 20 g de glycine, 1,5 g à 5 g de nucléotides, 0,5 g à 5 g de tributyrine, 5 g à 50 g de maltodextrine, 2 mg à 30 mg de β-carotène, 200 mg à 1 000 mg de vitamine E, 500 mg à 2 500 mg de vitamine C, 200 µg à 600 µg de sélénium et 10 mg à 30 mg de zinc.

17. Supplément selon l'une quelconque des revendications 1 à 16, **caractérisé en ce qu'**il est mis en oeuvre, en combinaison avec un aliment complet à administrer par voie parentérale en tant que système d'alimentation artificielle chez un sujet carencé, un malade critique ou chronique.

18. Supplément selon l'une quelconque des revendications 1 à 17, **caractérisé en ce qu'**il comprend en plus, par rapport à une dose journalière, une teneur en protéines allant jusqu'à 30 g et/ou une teneur en matières grasses allant jusqu'à 45 g et/ou en chrome allant jusqu'à 1 mg.

19. Supplément selon la revendication 18, **caractérisé en ce qu'**il contient les protéines supplémentaires sous forme d'un hydrolysat de protéine.

20. Supplément selon la revendication 18 ou 19, **caractérisé en ce qu'**il comprend les matières grasses supplémentaires sous forme d'acides gras à chaîne moyenne.

21. Supplément selon l'une quelconque des revendications 18 à 20, **caractérisé en ce que** les acides supplémentaires présentent une teneur élevée en acides gras n-3.

22. Supplément selon la revendication 21, **caractérisé en ce que** les acides gras n-3 contiennent de l'acide eicosapentanoïque et/ou de l'acide docosahexanoïque.

23. Supplément selon la revendication 21 ou 22, **caractérisé en ce que** la matière grasse comprend une huile de poisson ou un mélange de différentes huiles de poisson.

24. Supplément selon l'une quelconque des revendications 18 à 23, **caractérisé en ce qu'**il contient, par rapport à une dose journalière : 20 g à 45 g de glutamine, 5 g à 20 g de glycine, 1,5 g à 5 g de nucléotides, 0,5 g à 5 g de tributyrine, 5 g à 100 g de maltodextrine, 2 mg à 30 mg de β-carotène, 200 mg à 1 000 mg de vitamine E, 500 mg à 2 000 mg de vitamine C, 200 µg à 600 µg de sélénium, 10 mg à 30 mg de zinc, 200 µg à 600 µg de chrome, 15 g à 30 g d'hydrolysat de protéine, au total 15 g à 30 g de matières grasses supplémentaires, dont 7 g à 12 g d'acides gras à chaîne moyenne et 4 g à 8 g d'acides gras 3-n d'huile de poisson avec un rapport n-3/n-6 supérieur ou égal à 1:1, d'autres vitamines, oligo-éléments et minéraux.

25. Utilisation des composants a) à c) selon la revendication 1, pour la préparation d'un supplément à administrer par voie entérale pour le traitement de patients alimentés par voie parentérale sans contre-indication pour une introduction de substrat par voie entérale accompagné d'une intolérance élevée envers les solutions d'alimentation entérale classiques ou lors d'une intolérance moins prononcée envers les solutions d'alimentation entérale classiques, pour le traitement d'une alimentation carencée ou dans le cas d'une chirurgie abdominale lourde, pour le traitement de patients qui doivent rester à jeun avant des mesures diagnostiques ou avant des interventions chirurgicales, pour le traitement de maladies inflammatoires de l'intestin, de mucosites, de stomatites, du syndrome de l'intestin court et de la pancréatite aiguë.
